# EUROPEAN PATENT APPLICATION

(11) **EP 4 767 994 A1**
(43) Date of publication of application: **01.07.2026**
(21) Application number: 24867242.0
(22) Date of filing: 27.08.2024
(51) Int. Cl.: A61F 2/14

(54) **KERATOPROSTHESIS**

(30) Priority: 18.09.2023 CN 202311199721
(71) Applicant: Shanghai Vision Science Engineer Medical Equipment Co., Ltd., Shanghai 201201 (CN)
(72) Inventor: GUO, Yaxin, Shanghai 201201 (CN); JI, Lijun, Shanghai 201201 (CN); SUN, Junbao, Shanghai 201201 (CN)
(74) Representative: Raffay & Fleck
(86) International application number: PCT/CN2024/114735
(87) International publication number: WO 2025/060828

(57) **Abstract**

A keratoprosthesis (100), comprising a support (10) and a lens column (30), the lens column (30) comprising a body (31) and a collar (32). A middle part of the support (10) is provided with a mounting hole (10A), a hole wall of which being provided with an internal thread (111), and the outer peripheral wall of the collar (32) being provided with an external thread (321) fitting with the internal thread (111), so as to detachably assemble the support (10) and the collar (32). The material of the body (31) is polymethyl methacrylate, so as to provide a reliable light path structure, and the collar (32) is titanium metal. The body (31) and the collar (32) are integrally formed, such that the collar (32) is sleeved on the body (31) so as to form a lens column (30), and the outer peripheral wall of the body (31) is provided with a limiting slot (31D) for limiting the collar (32). The support (10) is titanium metal, such that the internal thread (111) and the external thread (321) are metal threads of the same material, and the heat shrinkage ratios of structures of the same material undergoing a temperature change are the same, so that a joint has a high assembly reliability and does not separate or fall off unless an external force is applied thereto. The service life of the artificial cornea is increased, surgical risks are reduced, a patient is protected from secondary injury or being blinded again, and use satisfaction is increased.

## Description

### CROSS-REFERENCES TO RELATED APPLICATIONS

The present application claims priority to Chinese Patent Application No. 202311199721.5 filed with the China National Intellectual Property Administration on September 18, 2023, entitled "Keratoprosthesis", which is hereby incorporated by reference in its entirety.

### FIELD

The present application relates to the technical field of medical devices, and in particular, to a keratoprosthesis.

### BACKGROUND

The human eye is an extremely complex and precise organ, and the transparent front 1/6 of the outermost layer of the eyeball wall is the well-known cornea, which plays an extremely important role in visual imaging. The world's first "World Report on Vision" released by the World Health Organization points out that more than 2.2 billion people worldwide suffer from visual impairment or blindness, and there are currently about 60 million patients with corneal blindness in the world. For visual impairment caused by corneal opacity due to various reasons, corneal transplantation is often the most effective rehabilitation method, and most corneal blindness patients may regain vision through conventional donor corneal transplantation.

However, due to factors such as the limited number of donor corneas, lack of corneal database information, and insufficient utilization of available corneas, the supply of corneal donors is still in a severely short state, and the development of keratoprostheses has always been the pursuit of scientists. Furthermore, corneal blindness patients who have suffered severe corneal thermal burns or chemical damage and have developed neovascularization are prone to rejection reactions after receiving donor corneal transplantation, leading to the failure of the vision restoration surgery.

The keratoprosthesis is a product similar to the human cornea made of medical polymer materials. In related art, the keratoprosthesis includes two parts of an optic cylinder and a stent. The optic cylinder is made of a transparent material with excellent optical properties and stable physicochemical properties, and is used to replace the opaque cornea that obstructs the optical pathway of the eyeball after lesion. The stent is a bridge used to connect the optic cylinder and surrounding tissues. Most keratoprostheses on the market currently consist of a stent made of a titanium plate or titanium foil and a polymethyl methacrylate (PMMA) optic cylinder. Long-term wear may cause the connection between the two to separate and detach, which not only invalidates the optical vision restoration function but may also cause secondary injury to the human eye.

### BRIEF SUMMARY

The main objective of the present application is to provide a keratoprosthesis, which aims to solve the technical problem that traditional titanium stents and PMMA optic cylinders are prone to detachment during long-term wear, leading to damage to human eye tissue or re-blindness.

To achieve the above objective, the keratoprosthesis proposed by the present application includes a stent and an optic cylinder;
the stent is made of a metal material, a central portion of the stent is provided with a mounting hole, a hole wall of the mounting hole is provided with an internal thread, and the optic cylinder is detachably provided in the mounting hole;
the optic cylinder includes a body and a sleeve, the body is made of polymethyl methacrylate, and the sleeve is made of a titanium metal;
the body is synthesized in-situ within the sleeve by photopolymerization and integrally formed with the sleeve, or the body is integrally formed with the sleeve by melt casting, and an outer peripheral wall of the body is provided with a limiting groove; and
the sleeve is sleeved at the body to form the optic cylinder, the sleeve is limited within the limiting groove, and an outer peripheral wall of the sleeve is provided with an external thread adapted to the internal thread.

In an embodiment of the present application, a diameter of the sleeve is 1.5 mm to 5 mm, and an axial length of the sleeve is 0.5 mm to 4 mm; and/or
a fixing hole is provided in a central portion of the sleeve, a hole wall of the fixing hole is provided with first limiting portions, and an outer peripheral wall of the body corresponding to a position of the fixing hole is provided with second limiting portions, where the second limiting portions are snap-fitted with the first limiting portions.

In an embodiment of the present application, a plurality of the first limiting portions are provided at interval, and the plurality of the first limiting portions correspond one-to-one to the second limiting portions; and/or
a plurality of the first limiting portions are provided, and the plurality of the first limiting portions are provided at intervals along a circumferential direction or an axial direction of the sleeve; and/or
the first limiting portions are distributed annularly along the circumferential direction of the sleeve; and/or
one of the first limiting portions and the second limiting portions is a concave groove, and the other of the two is a protrusion, where the protrusion is limited within the concave groove.

In an embodiment of the present application, the body includes an introductive segment, a fixing segment, an exporting segment, and an exposed segment connected in sequence along an axial direction;
an inwardly concave surface is provided at an end portion of the introductive segment away from the fixing segment;
the introductive segment and the exporting segment engage with an outer periphery of the fixing segment to form the limiting groove; and
an outwardly convex surface corresponding to the inwardly concave surface is provided at a side of the exposed segment facing away from the exporting segment.

In an embodiment of the present application, the inwardly concave surface is a spherical surface, and a spherical radius of the inwardly concave surface is 5 mm to 40 mm; and/or
the outwardly convex surface is a spherical surface, and a spherical radius of the outwardly convex surface is 5 mm to 9 mm; and/or
a diameter of the introductive segment is 1.5 mm to 5 mm, and an axial length of the introductive segment is 1 mm to 3 mm; and/or
a diameter of the exporting segment is 1.5 mm to 5 mm, and an axial length of the exporting segment is 1 mm to 4 mm; and/or
a diameter of the exposed segment is 1.5 mm to 11 mm, and an axial length of the exposed segment is 0.2 mm to 3 mm.

In an embodiment of the present application, the stent includes:
a connecting member, where the mounting hole is provided at a central portion of the connecting member;
a supporting member, where the supporting member is provided with a supporting cavity, and the connecting member is provided in the supporting cavity; and
at least two transition members, where the at least two transition members are provided in the supporting cavity at interval, and two ends of each of the transition members are respectively connected to the connecting member and the supporting member.

In an embodiment of the present application, a surface where the supporting member is located is an arcuate surface, the connecting member is provided corresponding to the arcuate surface, and a radius of curvature of the arcuate surface is infinitely close to a radius of curvature of a human corneal.

In an embodiment of the present application, the supporting member is annular; or
the supporting member is circular or elliptical.

In an embodiment of the present application, the keratoprosthesis further includes a plug, where an outer peripheral wall of the plug is provided with a threaded segment; and
the keratoprosthesis has a first state and a second state, where in the first state, the threaded segment of the plug is detachably assembled with the internal thread of the stent; and
in the second state, the external thread of the optic cylinder is detachably assembled with the internal thread of the stent.

The keratoprosthesis of the technical solution of the present application includes a stent and an optic cylinder. The optic cylinder includes a body for forming an optical path and a sleeve for connecting to the stent. A central portion of the stent is provided with a mounting hole, and a hole wall of the mounting hole is provided with an internal thread. An external thread adapted to the internal thread is provided at an outer peripheral wall of the sleeve, and the stent and the sleeve are detachably assembled through the internal thread and the external thread. The body is made of polymethyl methacrylate to provide a reliable optical path, and the sleeve is made of titanium metal. The body is synthesized in-situ within the sleeve by photopolymerization and integrally formed with the sleeve, or the body is integrally formed with the sleeve by melt casting. The sleeve is sleeved at the body to form the optic cylinder, an outer peripheral wall of the body is provided with a limiting groove, and the sleeve is limited within the limiting groove. Both the sleeve and the stent are made of titanium metal. The internal thread and the external thread are metal threads of the same material, the structure of the same material has the same thermal shrinkage ratio under temperature change, the assembly reliability at the connection is high, and no separation or detachment occurs without external force, which improves the service life of the keratoprosthesis, avoids secondary injury or re-blindness of the patient, and improves use satisfaction.

### BRIEF DESCRIPTION OF THE DRAWINGS

To more clearly illustrate the technical solutions in the embodiments of the present application, the accompanying drawings required for the description of the embodiments will be briefly introduced below. The accompanying drawings in the following description are merely some embodiments of the present application, and for those of ordinary skill in the art, other drawings may be obtained according to the structures shown in these drawings without creative efforts.
FIG. 1 is a schematic structural three-dimensional diagram of a keratoprosthesis according to an embodiment of the present application;
FIG. 2 is a schematic structural side view of a keratoprosthesis according to an embodiment of the present application;
FIG. 3 is a schematic structural three-dimensional diagram of an optic cylinder of a keratoprosthesis according to an embodiment of the present application;
FIG. 4 is a schematic structural front sectional view of an optic cylinder of a keratoprosthesis according to an embodiment of the present application;
FIG. 5 is a schematic structural three-dimensional diagram of a sleeve of a keratoprosthesis according to an embodiment of the present application;
FIG. 6 is a schematic structural top sectional view of a sleeve of a keratoprosthesis according to an embodiment of the present application;
FIG. 7 is a schematic structural three-dimensional diagram of a body of a keratoprosthesis according to an embodiment of the present application;
FIG. 8 is a schematic structural front sectional view of a body of a keratoprosthesis according to an embodiment of the present application;
FIG. 9 is a schematic structural three-dimensional diagram of a stent of a keratoprosthesis according to an embodiment of the present application; and
FIG. 10 is a schematic structural three-dimensional diagram of a plug of a keratoprosthesis according to another embodiment of the present application.

### Explanation of Reference Signs in the Drawings:

| Reference Signs | Name | Reference Signs | Name | Reference Signs | Name |
|---|---|---|---|---|---|
| 100 | keratoprosthesis | 30 | optic cylinder | 50 | plug |
| 10 | stent | 31 | body | 51 | threaded segment |
| 10A | mounting hole | 31A | inwardly concave surface | 52 | joining portion |
| 11 | connecting member | 31B | outwardly convex surface | 311 | fixing segment |
| 111 | internal thread | 31C | locking portion | 312 | introductive segment |
| 12 | supporting member | 32 | sleeve | 313 | exporting segment |
| 12A | supporting cavity | 321 | external thread | 314 | exposed segment |
| 13 | transition member | 322 | first limiting portion | 315 | second limiting portion |
| 32A | fixing hole | 323 | annular body | 31D | limiting groove |

The realization of the objective, functional features, and advantages of the present application will be further described in conjunction with the embodiments and with reference to the accompanying drawings.

### DETAILED DESCRIPTION

The technical solutions in the embodiments of the present application will be clearly and completely described below in conjunction with the accompanying drawings in the embodiments of the present application. The described embodiments are only a part of the embodiments of the present application, rather than all of the embodiments. All other embodiments obtained by those of ordinary skill in the art based on the embodiments in the present application without creative efforts shall fall within the protection scope of the present application.

It should be noted that all directional indications (such as upper, lower, left, right, front, rear...) in the embodiments of the present application are only used to explain the relative positional relationships and movement conditions between the components in a specific posture (as shown in the accompanying drawings). If the specific posture changes, the directional indications will also change accordingly.

In the present application, unless otherwise clearly specified and defined, the terms "connected", "fixed", etc. should be broadly understood. For example, "fixed" may be fixedly connected, detachably connected, or integrally formed, or it may be directly connected or indirectly connected through an intermediate medium, or it may be the internal communication between two elements or the mutual interaction relationship between two elements, unless otherwise clearly defined. For those of ordinary skill in the art, the specific meanings of the above terms in the present application may be understood according to specific circumstances.

In addition, in the present application, descriptions involving "first", "second", etc. are only for descriptive purposes, and should not be understood as indicating or implying relative importance or implicitly indicating the quantity of the indicated technical features. Thus, features defined by "first" and "second" may explicitly or implicitly include at least one such feature. Furthermore, the meaning of "and/or" appearing throughout the specification includes three parallel options. Taking "A and/or B" as an example, it includes option A, or option B, or option A and B simultaneously satisfied. Furthermore, the technical solutions between the various embodiments may be combined with each other, but such combination must be based on what may be achieved by those of ordinary skill in the art. When the combination of technical solutions is contradictory or unachievable, such combination of technical solutions should be considered non-existent and not within the claimed protection scope of the present application.

The human eye is an extremely complex and precise organ, and the transparent front 1/6 of the outermost layer of the eyeball wall is the well-known cornea, which plays an extremely important role in visual imaging. The world's first "World Report on Vision" released by the World Health Organization points out that more than 2.2 billion people worldwide suffer from visual impairment or blindness, and there are currently about 60 million patients with corneal blindness in the world. For visual impairment caused by corneal opacity due to various reasons, corneal transplantation is often the most effective rehabilitation method, and most corneal blindness patients may regain vision through conventional donor corneal transplantation.

However, due to factors such as the limited number of donor corneas, lack of corneal database information, and insufficient utilization of available corneas, the supply of corneal donors is still in a severely short state, and the development of keratoprostheses has always been the pursuit of scientists. Furthermore, corneal blindness patients who have suffered severe corneal thermal burns or chemical damage and have developed neovascularization are prone to rejection reactions after receiving donor corneal transplantation, leading to the failure of the vision restoration surgery.

The keratoprosthesis is a product similar to the human cornea made of medical polymer materials. In related art, the keratoprosthesis includes two parts of an optic cylinder and a stent. The optic cylinder is made of a transparent material with excellent optical properties and stable physicochemical properties, and is used to replace the opaque cornea that obstructs the optical pathway of the eyeball after lesion. The stent is a bridge used to connect the optic cylinder and surrounding tissues. Most keratoprostheses on the market currently consist of a stent made of a titanium plate or titanium foil and a PMMA optic cylinder. Long-term wear may cause the connection between the two to separate and detach, which not only invalidates the optical vision restoration function but may also cause secondary injury to the human eye.

The present application provides a keratoprosthesis. It should be noted that the human eye is an extremely complex and precise organ, and the transparent front 1/6 of the outermost layer of the eyeball wall is the well-known cornea. The cornea is mainly composed of avascular connective tissue, with a thickness of about 1 mm. The cornea is shaped like a convex-concave lens, has the function of refracting light, and plays an extremely important role in visual imaging. Both the stent 10 and the optic cylinder 30 of the keratoprosthesis 100 are safe bio-implant materials, which reduce post-operative rejection and facilitate the adherence and growth of human eye tissue to the stent 10 and the optic cylinder 30.

In the clinical surgical application of the traditional keratoprosthesis 100, the stent 10 is made of titanium metal or a titanium alloy, and the optic cylinder 30 is made of polymethyl methacrylate (PMMA). An internal thread 111 and an external thread 321 are correspondingly provided at the titanium metal stent 10 and the PMMA optic cylinder 30, respectively. The stent 10 and the optic cylinder 30 may be detachably connected. Due to the difference in materials, the toughness, brittleness, and thermal shrinkage ratio of the stent 10 and the optic cylinder 30 are all different. The keratoprosthesis 100 needs to remain in the patient's eye for several years or even more than ten years post-operatively. The connection between the PMMA optic cylinder 30 and the titanium metal stent 10 has different mechanical structures, and the thermal shrinkage ratio generated by heating also varies with temperature change, meaning the expansion coefficients of the different materials are different, which leads to uneven stress at the connection between the two different material components, resulting in displacement and squeezing at the connection between the two components. In mild cases, this partially affects the patient's vision restoration function and in severe cases, it causes the stent 10 and the optic cylinder 30 to detach and fall into the human eye tissue ultimately leading to secondary injury to the human eye, requiring re-operation, or, severely, damaging the eye tissue, and vision cannot be restored again.

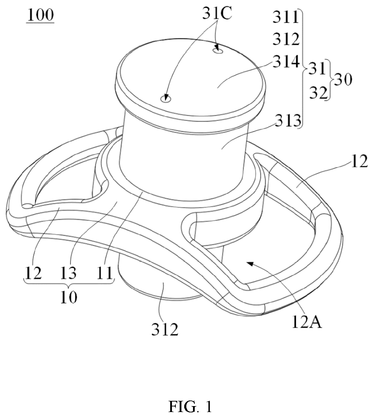

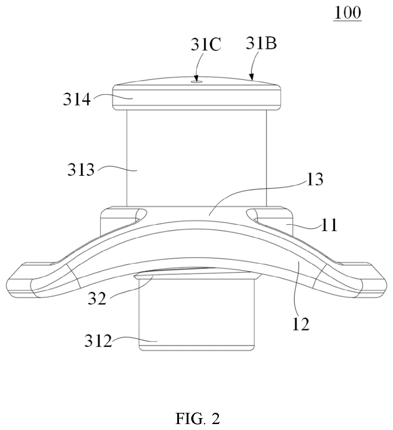

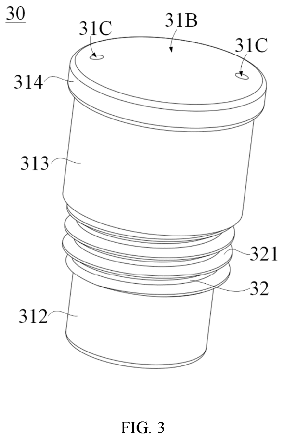

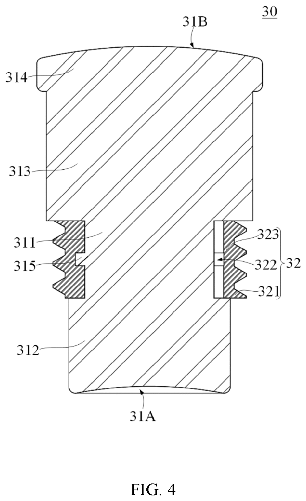

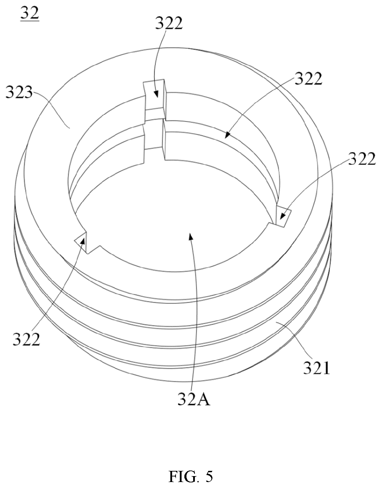

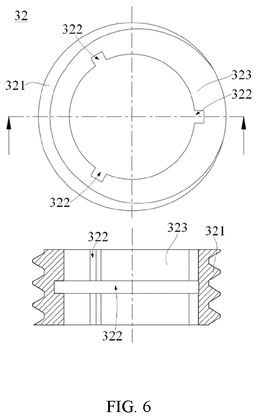

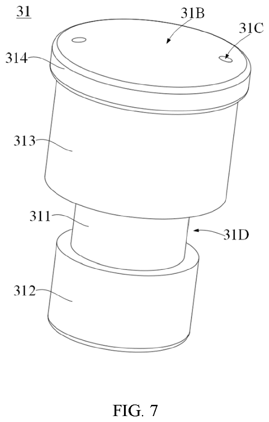

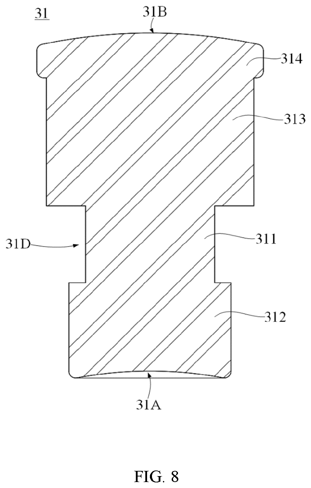

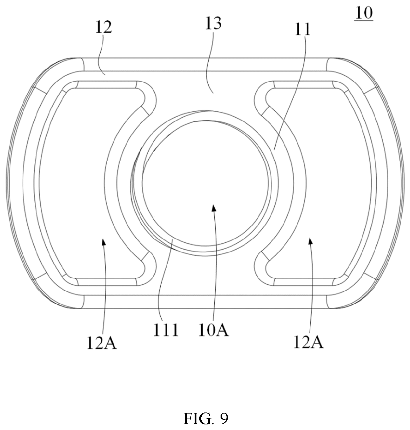

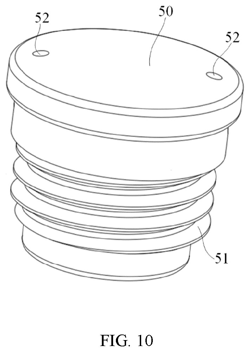

Referring to FIGS. 1 to 10, FIG. 1 is a schematic structural three-dimensional diagram of a keratoprosthesis 100 according to an embodiment of the present application; FIG. 2 is a schematic structural side view of a keratoprosthesis 100 according to an embodiment of the present application; FIG. 3 is a schematic structural three-dimensional diagram of an optic cylinder 30 of a keratoprosthesis 100 according to an embodiment of the present application; FIG. 4 is a schematic structural front sectional view of an optic cylinder 30 of a keratoprosthesis 100 according to an embodiment of the present application; FIG. 5 is a schematic structural three-dimensional diagram of a sleeve 32 of a keratoprosthesis 100 according to an embodiment of the present application; FIG. 6 is a schematic structural top sectional view of a sleeve 32 of a keratoprosthesis 100 according to an embodiment of the present application; FIG. 7 is a schematic structural three-dimensional diagram of a body of a keratoprosthesis 100 according to an embodiment of the present application; FIG. 8 is a schematic structural front sectional view of a body of a keratoprosthesis 100 according to an embodiment of the present application; FIG. 9 is a schematic structural three-dimensional diagram of a stent 10 of a keratoprosthesis 100 according to an embodiment of the present application; and FIG. 10 is a schematic structural three-dimensional diagram of a plug 50 of a keratoprosthesis 100 according to another embodiment of the present application.

In an embodiment of the present application, the keratoprosthesis 100 includes a stent 10 and an optic cylinder 30. The stent 10 is made of titanium metal. As shown in FIGS. 1 to 4 and FIG. 9, a central portion of the stent 10 is provided with a mounting hole 10A, and a hole wall of the mounting hole 10A is provided with an internal thread 111. The optic cylinder 30 is detachably provided in the mounting hole 10A. The optic cylinder 30 includes a body 31 and a sleeve 32. The body 31 is made of polymethyl methacrylate and the sleeve 32 is made of titanium metal. The body 31 is synthesized in-situ within the sleeve 32 by photopolymerization and integrally formed with the sleeve 32, or the body 31 is integrally formed with the sleeve 32 by melt casting. The sleeve 32 is sleeved at the body 31 to form the optic cylinder 30. The sleeve 32 is sleeved at the body 31, an outer peripheral wall of the body 31 is provided with a limiting groove 31D, the sleeve 32 is limited within the limiting groove 31D, and an outer peripheral wall of the sleeve 32 is provided with an external thread 321 adapted to the internal thread 111.

The keratoprosthesis 100 of the technical solution of the present application includes a stent 10 and an optic cylinder 30. The optic cylinder 30 includes a body 31 for forming an optical path and a sleeve 32 for connecting to the stent 10. A central portion of the stent 10 is provided with a mounting hole 10A, and a hole wall of the mounting hole 10A is provided with an internal thread 111. An outer peripheral wall of the sleeve 32 is provided with an external thread 321 adapted to the internal thread 111, and the stent 10 and the sleeve 32 are detachably assembled through the internal thread 111 and the external thread 321. The body 31 is synthesized in-situ within the sleeve 32 by photopolymerization and integrally formed with the sleeve 32, or the body 31 is integrally formed with the sleeve 32 by melt casting. The sleeve 32 is sleeved at the body 31 to form the optic cylinder 30, and in the axial direction of the optic cylinder 30, the two ends of the body 31 protrude from the two ends of the sleeve 32. An outer peripheral wall of the formed body 31 is provided with a limiting groove 31D, and the sleeve 32 is limited within the limiting groove 31D. The body 31 of the keratoprosthesis 100 of the present application is made of polymethyl methacrylate to provide a reliable optical path. Both the sleeve 32 and the stent 10 are made of titanium metal. The internal thread 111 and the external thread 321 are metal threads of the same material. The structure of the same material has the same thermal shrinkage ratio under temperature change, the assembly reliability at the connection is high, and no separation or detachment occurs without external force, which improves the service life of the keratoprosthesis 100, avoids secondary injury or re-blindness of the patient, and improves use satisfaction.

Compared with the related art, the assemblable fixed-wing keratoprosthesis 100 designed by the present application is essentially a special refractive device assembled manually, which may be used to replace the opaque cornea that affects the optical pathway of the eyeball after lesion, and enables the patient to regain vision by exerting its function as a transparent refractive medium. Due to the good biocompatibility of the materials used, it is easy to heal with the surrounding tissue and is not prone to post-transplantation rejection. It is suitable for patients with bilateral opaque blindness who have failed conventional corneal transplantation, including patients with severe chemical burns, severe corneal neovascularization caused by various etiologies, and severe dry eyes.

Furthermore, the external thread 321 of the sleeve 32 engages with the internal thread 111 of the stent 10 to fix the entire optic cylinder 30, resulting in stronger structural stability and also allowing for effective sealing, to prevent leakage of intraocular fluid. Using the medical titanium metal sleeve 32 to engage with the inner ring of the stent 10 will be effectively tighter and less prone to detachment during actual assembly, reducing various unexpected risks during surgical implantation, improving the success rate of post-operative recovery, and allowing for the adjustment of the relative position of the optic cylinder 30 within a certain range.

Furthermore, the material used for the body 31 of the optic cylinder 30 is polymethyl methacrylate, abbreviated as PMMA. Polymethyl methacrylate is a transparent, carbon-containing, high-molecular-weight, inert organic substance to which corneal tissue has good tolerance. It has high light transmittance and a high refractive index, stable performance, high resistance to aging and environmental changes, no biodegradability, resistance to acids, alkalis, and organic solvents, light weight, is not easy to break, has strong plasticity, and is easy to process.

In an embodiment of the present application, the body 31 is synthesized in-situ within the sleeve 32 by photopolymerization and integrally formed with the sleeve 32 to form the optic cylinder 30. Alternatively, the body 31 is integrally formed with the sleeve 32 by melt casting to form the optic cylinder 30.

Furthermore, in the structure of the keratoprosthesis 100 of the present application, the optic cylinder 30 is columnar, and a radial cross-section of the optic cylinder 30 is circular. The sleeve 32 and the body 31 forming the optic cylinder 30 are integrally formed. The integral form may be pre-manufactured and then the external thread 321 at the outer periphery of the sleeve 32 of the integral optic cylinder 30 and the internal thread 111 of the stent 10 are thread-assembled to achieve detachable assembly of the optic cylinder 30 and the stent 10.

In manufacturing, the sleeve 32 is an annular structure made of titanium metal. A fixing hole 32A may be provided at a central portion of the annular structure. The pre-manufactured sleeve 32 is placed in a photopolymerization mold or a casting mold. The body 31 is synthesized in-situ within the sleeve 32 by photopolymerization and integrally formed with the sleeve 32 into the optic cylinder 30, or the body 31 is integrally formed with the sleeve 32 by melt casting to form the optic cylinder 30.

The raw material for manufacturing the body 31 is placed in a mold pre-loaded with the sleeve 32. Through photopolymerization and curing, the body 31 is formed in the fixing hole 32A of the sleeve 32 and continues to be synthesized along the axial extension direction from the fixing hole 32A. Both ends of the body 31 protrude from both axial ends of the sleeve 32.

In the melt casting mold, the sleeve 32 is pre-placed inside the mold, and then the raw material of the body 31 is melted into a liquid state and injected into the mold. The liquid raw material of the body 31 fills the molding cavity of the mold to form the body 31 partially penetrating through the fixing hole 32A of the sleeve 32 and extending in opposite directions at both ends, to integrally form the sleeve 32 and the body 31 of different materials, facilitating the detachable assembly of the external thread 321 of the titanium metal sleeve 32 with the internal thread 111 of the same material stent 10.

The integral pre-forming of the sleeve 32 and the body 31 is achieved through photopolymerization or melt casting. The light may be ultraviolet light which enhances the structural stability of the optic cylinder 30 itself. The sleeve 32 and the body 31 of different materials do not relatively displace from each other, improving the structural stability of the optic cylinder 30 itself. The threaded structures of the same material have the same coefficient of expansion under temperature change. The connection will not suffer from uneven stress caused by thermal expansion, which enhances the structural stability of the assembly of the optic cylinder 30 and the stent 10.

The axial extension direction of the optic cylinder 30 and the stent 10 is defined as the length direction, and the radial extension direction is defined as the thickness direction. In clinical medical application, the structural precision of the keratoprosthesis 100 corresponding to the human cornea area is relatively fine and the volume is small. To facilitate clinical surgery and post-operative wound suturing, the radial cross-sectional area of the stent 10 of the keratoprosthesis 100 is smaller than the area of the human iris area.

It should be noted that the sleeve 32 and the body 31 are provided as an integral structure where a fixing hole 32A is formed at the central portion of the sleeve 32. The portion of the body 31 formed inside the fixing hole 32A not only enhances the reliability of the integral forming of the sleeve 32 and the body 31, but also ensures that the sleeve 32 has a certain thickness along its radial direction. However, the thickness of the portion of the stent 10 connecting the sleeve 32 after removing the position of the mounting hole 10A is also limited. Therefore, forming the internal thread 111 or the external thread 321 at the small-volume, high-precision component of the keratoprosthesis 100 is a high-difficulty operation, which is difficult to achieve with the precision of traditional device.

In an embodiment of the present application, a diameter of the sleeve 32 is 1.5 mm to 5 mm, and an axial length of the sleeve 32 is 0.5 mm to 4 mm.

In an embodiment of the present application, the external thread 321 at the sleeve 32 and the internal thread 111 at the stent 10 are formed by a milling process. The sleeve 32 is made of titanium metal. The axial length of the sleeve 32 is 0.8 mm to 4 mm, the diameter of the sleeve 32 is 1.5 mm to 5 mm, and the thickness of the sleeve 32 is 0.3 mm to 0.8 mm. The size of the sleeve 32 is customized according to the patient's condition and is not uniquely limited here.

By way of example, taking the maximum diameter of the sleeve 32 as 5 mm, assuming that a fixing hole 32A with an aperture of 3.5 mm is formed inside the sleeve 32, then the annular wall thickness of the sleeve 32 is about 0.8 mm, and the external thread 321 is formed at the outer wall of the hole with a thickness of 0.8 mm on by a milling process. The precision and difficulty of the milling process are relatively high, and the device used is also high-precision and high-cost device.

The thickness of the portion of the stent 10 connecting the sleeve 32 after removing the mounting hole 10A may be provided as 0.1 mm to 2 mm, where the aperture of the mounting hole 10A may be provided corresponding to the outer diameter of the sleeve 32, i.e., the aperture of the mounting hole 10A may be 1.5 mm to 5 mm. Taking a sleeve 32 where the maximum thickness of the stent 10 is 2 mm as an example, the internal thread 111 is formed at the inner wall of the hole with a thickness of 2 mm in the mounting hole 10A with an aperture of 5 mm at the stent 10 by a milling process. While forming the thread of the same material at the outer peripheral side of the sleeve 32, the structural stability of the sleeve 32 must be ensured and the mechanical structural balance at the connection between the two may also be ensured, extending the post-operative service life of the keratoprosthesis 100.

The preparation of the components of the above-mentioned keratoprosthesis 100 has higher requirements for the processing head, processing precision, processing control, and operating technology for the internal thread 111 or external thread 321 processing of the milling device than those of the traditional keratoprosthesis 100. The manufacturing cost is high, and device that meets the requirements may improve the yield of component manufacturing and extend the service life of the keratoprosthesis 100 after component assembly.

Referring to FIGS. 4 to 6, in an embodiment of the present application, a fixing hole 32A is provided in a central portion of the sleeve 32, a hole wall of the fixing hole 32A is provided with first limiting portions 322, and an outer peripheral wall of the body 31 corresponding to a position of the fixing hole 32A is provided with second limiting portions 315, where the second limiting portions 315 are snap-fitted with the first limiting portions 322.

In this embodiment, the sleeve 32 includes an annular body 323, a fixing hole 32A is provided in a central portion of the annular body 323, a central portion of the body 31 penetrates through the fixing hole 32A, and the two ends of the body 31 respectively extend out from the two axial ends of the sleeve 32. The contact surface of the sleeve 32 corresponding to the body 31 is provided with the first limiting portions 322, and the first limiting portions 322 are formed at the hole wall of the fixing hole 32A in the central portion of the annular body 323. An outer peripheral wall of the body 31 corresponding to the connection with the sleeve 32 are provided with the second limiting portions 315, and the second limiting portions 315 are snap-fitted with the first limiting portions 322, which improves the connection reliability between the sleeve 32 and the body 31, and may also be used to restrict the relative displacement of the body 31 and the sleeve 32 in the axial direction or the radial circumferential direction. The integral optic cylinder 30 has high structural stability, is resistant to external force twisting or pulling, and has a long service life.

It may be understood that a radial cross-section of the optic cylinder 30 is circular. In the respective cross-sections of the body 31 and the sleeve 32, the respective radial cross-sections of the body 31 extending along the axial direction are a plurality of circles of unequal diameter specifications, and the cross-sections of the sleeve 32 are annular. Since the overall structure of the keratoprosthesis 100 has a small volume, the influence of the first limiting portions 322 and the second limiting portions 315 on the cross-sectional shapes of the two may be considered negligible.

In an embodiment of the present application, a plurality of the first limiting portions 322 are provided at interval, and the plurality of first limiting portions 322 correspond one-to-one to the second limiting portions 315; and/or
a plurality of the first limiting portions 322 are provided, and the plurality of the first limiting portions 322 are provided at intervals along a circumferential direction or an axial direction of the sleeve 32; and/or
the first limiting portions 322 are distributed annularly along the circumferential direction of the sleeve; and/or
one of the first limiting portions 322 and the second limiting portions 315 is a concave groove, and the other of the two is a protrusion, where the protrusion is limited within the concave groove.

In this embodiment, the first limiting portions 322 correspond one-to-one to the second limiting portions 315, where the correspondence includes the correspondence of the quantity, shape, and setting position of the first limiting portions 322 and the second limiting portions 315.

Based on the above-mentioned correspondence, in an embodiment, one of the first limiting portions 322 and the second limiting portions 315 is a concave groove, and the other of the two is a protrusion, and the protrusion is limited within the concave groove to enhance the connection strength between the sleeve 32 and the body 31. For example, the inner side of the fixing hole 32A of the sleeve 32 is provided with concave grooves as the first limiting portions 322 and the outer periphery of the body 31 is provided with protrusions as the second limiting portions 315, the concave grooves may be dotted grooves, straight grooves, curved segment grooves, or an annular groove connected end-to-end, and the protrusions and the concave grooves are adaptively set.

Based on the structural arrangement of the protrusions and the concave grooves in the above embodiment, the first limiting portions 322 in another embodiment may be provided as one or more. When the concave groove is provided as one, the concave groove may be a dotted groove, a straight groove, a curved segment groove, or an annular groove connected end-to-end; and when the concave grooves are provided as plural, the concave grooves may be a plurality of individual dotted grooves, straight grooves, curved segment grooves, or annular grooves connected end-to-end or may be any free combination of the above groove type, and a plurality of protrusions are adaptively provided with a plurality of concave grooves, which will not be exhausted one by one here.

In yet another embodiment, the first limiting portions 322 and the second limiting portions 315 are respectively provided as plural. The concave grooves of the plurality of first limiting portions 322 are distributed in a dotted or annular arrangement, and may be regularly or irregularly distributed, as long as that the connection stability between the sleeve 32 and the body 31 is improved.

Furthermore, one end of the body 31 extends into the human eye and is provided with an inwardly concave surface 31A, and the other end is exposed at the iris position and is provided with an outwardly convex surface 31B. The inwardly concave surface 31A and the outwardly convex surface 31B are embodied on the body 31 as curved surfaces formed by bending on the same side. The inwardly concave surface 31A engaging with the outwardly convex surface 31B may give the body 31 of the optic cylinder 30 a certain refractive power, increase the light collection angle, expand the patient's field of view, and also play a role in correcting vision.

Referring to FIGS. 3, 4, 7 and 8, in an embodiment of the present application, the body 31 includes an introductive segment 312, a fixing segment 311, an exporting segment 313, and an exposed segment 314 connected in sequence along an axial direction. An inwardly concave surface 31A is provided at an end portion of the introductive segment 312 away from the fixing segment 311, the introductive segment 312 and the exporting segment 313 engage with an outer periphery of the fixing segment 311 to form the limiting groove 31D, and an outwardly convex surface 31B corresponding to the inwardly concave surface 31A is provided at a side of the exposed segment 314 facing away from the exporting segment 313.

In this embodiment, the body 31 includes an introductive segment 312, a fixing segment 311, an exporting segment 313, and an exposed segment 314 connected in sequence along an axial direction. The fixing segment 311 is used to connect to the sleeve 32. An end portion of the introductive segment 312 is provided with an inwardly concave surface 31A, and an end portion of the exposed segment 314 is provided with an outwardly convex surface 31B. The inwardly concave surface 31A and the outwardly convex surface 31B are located at the two ends of the body along the radial direction. The setting of the two curved surfaces gives the optic cylinder 30 a certain refractive power, increases the light collection angle, expands the patient's field of view, and also plays a role in correcting vision.

An outer peripheral wall of the body 31 is provided with a limiting groove 31D, and the sleeve 32 is limited within the limiting groove 31D. In an embodiment, the outer peripheral wall of the body 31 is provided with the limiting groove 31D for limiting the sleeve 32, the limiting groove 31D is formed by the introductive segment 312 and the exporting segment 313 engaging with the outer periphery of the fixing segment 311, and the limiting groove 31D is presented as an annular groove recessed inward along the radial direction of the body 31. The sleeve 32 is embedded in the annular limiting groove 31D, and an external thread 321 is provided on the outer peripheral wall of the sleeve 32. After the sleeve 32 and the body 31 are integrally formed, an outer diameter of the sleeve 32 is greater than or equal to an outer diameter of the introductive segment 312, and an outer diameter of the exporting segment 313 is greater than the outer diameter of the sleeve 32. A step is formed at the connection between the sleeve 32 and the exporting segment 313. This step is used to limit the threading stroke of the sleeve 32 and the stent 10 to prevent the threaded connection of the optic cylinder 30 and the stent 10 from slipping relative to each other, improving the connection reliability of the optic cylinder 30 and the stent 10.

It may be understood that the side of the exposed segment 314 facing the outside is provided with a locking portion 31C matching an external assembly tool, and the locking portion 31C is located at the edge of the exposed segment 314, which facilitates disassembly and assembly.

In an embodiment of the present application, the inwardly concave surface 31A is a spherical surface, and a spherical radius of the inwardly concave surface 31A is 5 mm to 40 mm; and/or
the outwardly convex surface 31B is a spherical surface, and a spherical radius of the outwardly convex surface 31B is 5 mm to 9 mm; and/or
a diameter of the introductive segment 312 is 1.5 mm to 5 mm, and an axial length of the introductive segment 312 is 1 mm to 3 mm; and/or
a diameter of the exporting segment 313 is 1.5 mm to 5 mm, and an axial length of the exporting segment 313 is 1 mm to 4 mm; and/or
a diameter of the exposed segment 314 is 1.5 mm to 11 mm, and an axial length of the exposed segment 314 is 0.2 mm to 3 mm.

In this embodiment, generally, the inwardly concave surface 31A is a spherical surface, and the outwardly convex surface 31B is a spherical surface. The inwardly concave surface 31A and the outwardly convex surface 31B of the optic cylinder 30 determine the refractive power of the optic cylinder 30, and also determine the patient's post-operative field of view. The inwardly concave surface 31A and the outwardly convex surface 31B are located at the two ends of the body along the radial direction to give the optic cylinder 30 a certain refractive power, increase the light collection angle, expand the patient's field of view, and also play a role in correcting vision.

In this embodiment, the present application designs the body of the optic cylinder 30 to include a fixing segment 311, an introductive segment 312, an exporting segment 313 and an exposed segment 314.

The use of a medical titanium metal or titanium alloy sleeve 32 engaging with the stent 10 will be tighter and less prone to detachment during actual assembly, and may also adjust the relative position of the optic cylinder 30 within a certain range.

An outer diameter of the introductive segment 312 is less than or equal to an aperture of the mounting hole 10A of the stent 10, which allows the optic cylinder 30 to quickly enter the mounting hole 10A of the stent 10.

The external thread 321 of the sleeve 32 assembled to the optic cylinder 30 engages with the internal thread 111 of the stent 10, to achieve the function of fixing the optic cylinder 30 and the stent 10, and may also perform effective sealing to prevent leakage of intraocular fluid.

The exporting segment 313 forms a part of the optical path of the optic cylinder 30, realizing effective adjustment of externally collected light.

The exposed segment 314 is umbrella-shaped, and an outer diameter of the exposed segment 314 is greater than an outer diameter of the exporting segment 313, realizing effective collection of external light, and also preventing human eye tissue from accumulating around the exporting segment 313 to affect light transmittance, and improving light transmittance and avoiding secondary injury during adjustment and replacement of the optic cylinder 30.

In implementation, a spherical radius of the inwardly concave surface 31A is 9.5 mm to 40 mm.

Based on the inwardly concave surface 31A, the outwardly convex surface 31B is a spherical surface, and a spherical radius of the outwardly convex surface 31B is 5 mm to 9 mm.

Based on the outwardly convex surface 31B and the inwardly concave surface 31A, a diameter of the introductive segment 312 is 1.5 mm to 5 mm, and an axial length of the introductive segment 312 is 1 mm to 3 mm.

Based on the outwardly convex surface 31B, the inwardly concave surface 31A, and the introductive segment 312, a diameter of the exporting segment 313 is 1.5 mm to 5 mm, and an axial length of the exporting segment 313 is 1 mm to 4 mm.

Based on the outwardly convex surface 31B, the inwardly concave surface 31A, the introductive segment 312, and the exporting segment 313, a diameter of the exposed segment 314 is 1.5 mm to 11 mm, and an axial length of the exposed segment 314 is 0.2 mm to 3 mm.

It may be understood that the sizes of the above-mentioned component parts are provided corresponding to the patient's needs, and the sizes of the respective parts may be provided independently or in combination with each other so as to expand the scope of application of the keratoprosthesis 100.

In an embodiment of the present application, the stent 10 includes a connecting member 11, a supporting member 12, and at least two transition members 13. The mounting hole 10A is provided in a central portion of the connecting member 11. The supporting member 12 is provided with a supporting cavity 12A. The connecting member 11 is provided in the supporting cavity 12A. The at least two transition members 13 are provided in the supporting cavity 12A at interval, and two ends of each of the transition members 13 are respectively connected to the supporting member 12 and the connecting member 11.

In this embodiment, the stent 10 of the keratoprosthesis 100 may be hollowed-out, or in the shape of side teeth arranged in parallel with each other. Taking the hollowed-out stent 10 as an example, the connecting member 11 may serve as an inner annular segment of the stent 10. The inner annular segment is used to connect the sleeve 32. The supporting member 12 serves as an outer annular segment of the stent 10. The outer annular segment and the inner annular segment are connected by at least two connecting members 11. The at least two connecting members 11 are transition bridges. The arrangement of the transition bridges allows the space between the inner annular member and the outer annular member to be hollowed-out, which not only reduces the weight of the stent 10, but also provides a hollowed-out outer supporting ring for the adherence and growth of the eye tissue to the stent 10, enhancing the structural stability of the stent 10 when placed in the human eye, and facilitating post-operative growth.

Furthermore, the stent 10 is connected by two or more transition bridges between the inner and outer annular structures. The maximum transverse diameter may be 8.4 mm. The outer annular segment of the stent 10 facilitates fixation within the cornea. When a lamellar incision is made, the separation of the corneal pocket is reduced, which reduces damage.

The medical titanium metal or medical titanium alloy material used for the stent 10 serves as a safe bio-implant material with high biological safety, and the titanium surface is simultaneously modified with hydroxyapatite or bioactive glass. When the stent 10 is implanted in the human body, the first occurrence is the adsorption of biological macromolecules in the tissue fluid on the surface of the implant, which guides cell adhesion, influences cell growth, differentiation, and functional expression, and ultimately completes tissue healing.

In an embodiment of the present application, a surface where the supporting member 12 is located is an arcuate surface, the connecting member 11 is provided corresponding to the arcuate surface, and a radius of curvature of the arcuate surface is infinitely close to a radius of curvature of a human corneal.

In this embodiment, the structural design of the overall stent 10 is beneficial to the adhesion of corneal cells and matrix. When the human eye tissue grows, the tissue around the stent 10 may grow together, fixing the stent 10 more firmly and enhancing its positional stability. Moreover, the radius of curvature of the supporting member 12 of the outer ring of the stent 10 is close to the radius of curvature of the human cornea, and this curvature reduces the surgical difficulty during implantation and the mechanical pressure exerted by the peripheral part of the titanium stent 10 on the surrounding corneal tissue. The relatively thin thickness of the stent 10 does not affect the nutritional metabolism and transport between the corneal layers.

In an embodiment of the present application, the supporting member 12 is annular, or the supporting member 12 is circular or elliptical.

In this embodiment, the supporting member 12 is circular, that is, the supporting cavity 12A is circular, and the connecting member 11 is located at the center of the supporting member 12, which facilitates the placement of the keratoprosthesis 100 in the human eye, eliminates the need for subsequent adjustment of the circumferential position, and improves surgical convenience.

In another embodiment, the supporting member 12 is elliptical. The elliptical shape is similar to the shape of a sports field having a long-side direction and a short-side direction. In a clinical operations, the short-side direction faces the wound location, which reduces the supporting force of the stent 10 on the iris tissue of the human eye, facilitates post-operative suturing, and accelerates post-operative recovery.

Referring to FIG. 10, in an embodiment of the present application, the keratoprosthesis 100 further includes a plug 50, and an outer peripheral wall of the plug 50 is provided with a threaded segment 51. The keratoprosthesis 100 has a first state and a second state. In the first state, the threaded segment 51 of the plug 50 is detachably assembled with the internal thread 111 of the stent 10, and in the second state, the external thread 321 of the optic cylinder 30 is detachably assembled with the internal thread 111 of the stent 10.

In this embodiment, the keratoprosthesis 100 provided by the present application is an assemblable fixed-wing keratoprosthesis 100. The optic cylinder 30 or the plug 50 is selectively assembled with the stent 10. The implantation of the keratoprosthesis 100 into the human body is carried out in two stages. In the first stage, the fixed-wing stent 10 is implanted between the corneal layers, and the opening is sealed by assembling the plug 50. In the second stage, the plug 50 is removed after a period of time (post-operative growth and recovery of the stent 10 and human eye tissue), and the threaded optic cylinder 30 is implanted into the mounting hole 10A of the inner annular segment of the stent 10. Dividing the surgery into two stages allows for observation of the condition of the stent 10 after the first stage of implantation and checking for complications. In case of infection or corneal melting and thinning, anti-inflammatory treatment and various strengthening measures may be performed on the cornea, and the tissue is allowed to heal firmly over a relatively long period of time.

Furthermore, the plug 50 may be made of PMMA material or titanium metal material or may be integrally formed from PMMA material and titanium metal material with reference to the manufacturing method of the optic cylinder 30. A side of the plug 50 facing the outside is provided with a joining portion 52 matching an external assembly tool, which facilitates disassembly and assembly.

The foregoing describes only the preferred embodiments of the present application and should not therefore limit the patent scope of the present application. Any equivalent structural changes made under the inventive concept of the present application utilizing the content of the description and drawings of the present application or directly/indirectly applied to other related technical fields, shall be included within the patent protection scope of the present application.

## Claims

1. A keratoprosthesis, wherein the keratoprosthesis comprises a stent and an optic cylinder;
the stent is made of a metal material, a central portion of the stent is provided with a mounting hole, a hole wall of the mounting hole is provided with an internal thread, and the optic cylinder is detachably provided in the mounting hole;
the optic cylinder comprises a body and a sleeve, the body is made of a transparent polymer material, and the sleeve is made of a metal material;
the body and the sleeve are integrally formed, and an outer peripheral wall of the body is provided with a limiting groove; and
the sleeve is sleeved at the body to form the optic cylinder, the sleeve is limited within the limiting groove, and an outer peripheral wall of the sleeve is provided with an external thread adapted to the internal thread.

2. The keratoprosthesis of claim 1, wherein the stent is made of titanium metal or a titanium alloy, the body is made of polymethyl methacrylate, and the sleeve is made of titanium metal or a titanium alloy.

3. The keratoprosthesis of claim 1, wherein the body is synthesized in-situ within the sleeve by photopolymerization and integrally formed with the sleeve, or the body is integrally formed with the sleeve by melt casting.

4. The keratoprosthesis of claim 1, wherein a diameter of the sleeve is 1.5 mm to 5 mm, and an axial length of the sleeve is 0.5 mm to 4 mm; and/or
a fixing hole is provided in a central portion of the sleeve, a hole wall of the fixing hole is provided with first limiting portions, and an outer peripheral wall of the body corresponding to a position of the fixing hole is provided with second limiting portions, wherein the second limiting portions are snap-fitted with the first limiting portions.

5. The keratoprosthesis of claim 4, wherein a plurality of the first limiting portions are provided at interval, and the plurality of the first limiting portions correspond one-to-one to the second limiting portions; and/or
a plurality of the first limiting portions are provided, and the plurality of the first limiting portions are provided at intervals along a circumferential direction or an axial direction of the sleeve; and/or
the first limiting portions are distributed annularly along the circumferential direction of the sleeve; and/or
one of the first limiting portions and the second limiting portions are concave grooves, and the other of the two are protrusions, wherein the protrusions are limited within the concave grooves.

6. The keratoprosthesis of claim 1, wherein the body comprises an introductive segment, a fixing segment, an exporting segment, and an exposed segment connected in sequence along an axial direction;
an inwardly concave surface is provided at an end portion of the introductive segment away from the fixing segment;
the introductive segment and the exporting segment engage with an outer periphery of the fixing segment to form the limiting groove; and
an outwardly convex surface corresponding to the inwardly concave surface is provided at a side of the exposed segment facing away from the exporting segment.

7. The keratoprosthesis of claim 6, wherein the inwardly concave surface is a spherical surface, and a spherical radius of the inwardly concave surface is 5 mm to 40 mm; and/or
the outwardly convex surface is a spherical surface, and a spherical radius of the outwardly convex surface is 5 mm to 9 mm; and/or
a diameter of the introductive segment is 1.5 mm to 5 mm, and an axial length of the introductive segment is 1 mm to 3 mm; and/or
a diameter of the exporting segment is 1.5 mm to 5 mm, and an axial length of the exporting segment is 1 mm to 4 mm; and/or
a diameter of the exposed segment is 1.5 mm to 11 mm, and an axial length of the exposed segment is 0.2 mm to 3 mm.

8. The keratoprosthesis of any of claims 1 to 7, wherein the stent comprises:
a connecting member, wherein the mounting hole is provided at a central portion of the connecting member;
a supporting member, wherein the supporting member is provided with a supporting cavity, and the connecting member is provided in the supporting cavity; and
at least two transition members, wherein the at least two transition members are provided in the supporting cavity at interval, and two ends of each of the transition members are respectively connected to the connecting member and the supporting member.

9. The keratoprosthesis of claim 8, wherein a surface where the supporting member is located is an arcuate surface, the connecting member is provided corresponding to the arcuate surface, and a radius of curvature of the arcuate surface is infinitely close to a radius of curvature of a human corneal.

10. The keratoprosthesis of claim 8, wherein the supporting member is annular; or
the supporting member is circular or elliptical.

11. The keratoprosthesis of any of claims 1 to 7, wherein the keratoprosthesis further comprises a plug, wherein an outer peripheral wall of the plug is provided with a threaded segment; and
the keratoprosthesis has a first state and a second state, wherein in the first state, the threaded segment of the plug is detachably assembled with the internal thread of the stent; and
in the second state, the external thread of the optic cylinder is detachably assembled with the internal thread of the stent.
